Europäisches Patentamt

European Patent Office

Office européen des brevets

(11) Publication number: **0 464 300 A2**

# EUROPEAN PATENT APPLICATION

(21) Application number: **91100027.1**

(22) Date of filing: **02.01.91**

(51) Int. Cl.5: **C07K 3/02**, C07K 15/10, A61K 35/78, A61K 37/64

(30) Priority: **04.07.90 JP 175185/90**

(43) Date of publication of application:
**08.01.92 Bulletin 92/02**

(84) Designated Contracting States:
**DE FR GB IT SE**

(71) Applicant: **Kyodo Milk Industry Corporation Limited**
**17-2, Koami-cho Nihonbashi**
**Chuo-ku Tokyo(JP)**

(72) Inventor: **Oishi, Hifumi**
**No.303, 27-6, Sekimachi-higashi 1-chome**
**Nerima-ku, Tokyo(JP)**

Inventor: **Hattori, Takashi**
**No.205, 1-19, Jyosuihoncho 5-chome**
**Kodaira-shi, Tokyo(JP)**
Inventor: **Watanabe, Masatoshi**
**5-36, Midoricho 2-chome**
**Koganei-shi, Tokyo(JP)**
Inventor: **Kato, Akio**
**24-28, Kitamachi 4-chome**
**Kokubunji-shi, Tokyo(JP)**

(74) Representative: **Lesser, Karl-Bolko, Dipl.-Ing.**
**Patentanwalt & European Patent Attorney**
**Gnesener Strasse 2**
**W-8068 Pfaffenhofen 1(DE)**

(54) Process for producing alpha-amylase inhibitor derived from wheat seed.

(57) A method of extracting α-amylase inhibitor not containing enzymes simply and at high yield with 58 to 70 % ethanol from powderized products of wheat or the like.

Fig. 1

Background of the invention

Field of the invention

Use of an α-amylase inhibitor (hereinafter simply referred to as α-AI) is extremely useful for inhibiting of absorption of starch substance from intestinal tracts and there has been considered various application use therefore, such as obesitic or diabetic diet, and the present invention concerns with a simple process for producing the α-AI.

There have been made detailed studies on α-AI in the seeds of wheat or the like and several production processes have been reported. In any of the reported processes for producing α-Ai, steps of extraction, salting out, ion exchange and gel filtration are combined.

However, when production of α-AI is tried in an industrial scale by the known process, it has been found that it is not practical to use α-AI with the foregoing object since the production cost is extremely high and the recovery yield is very low. In view of the above, the present inventors have attempted a development for the simple production process of α-AI and achieved a simple process for producing α-AI not containing enzymes such as α-amylase or lipase, at a high yield with simple steps of combining ethanol extraction and ultra-filtration.

Summary of the invention

The foregoing object of the present invention can be attained by a method of extracting α-AI from powderization product of wheat or the like with 58 - 70 % ethanol, as well as a powderization process for α-AI using a spray-drying step under the conditions of an inlet temperature at 90 to 110 °C and the exit temperature at 40 to 50 °C, respectively.

Description of the accompanying drawings

Fig. 1    is a graph illustrating the conditions for extracting α-AI with ethanol; and

Fig. 2    is a graph illustrating the purity of α-AI.

Example

Description will then be made in details for the simple process for producing α-AI.

(1) Extraction conditions for α-AI.

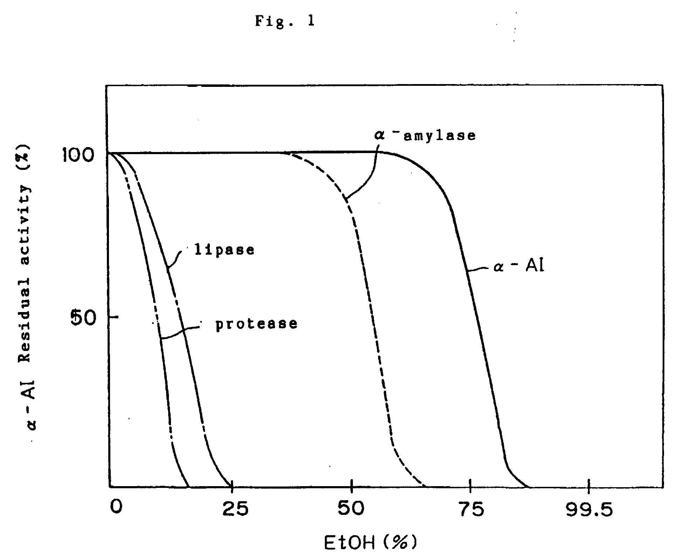

α-AI was extracted from a delipidated wheat powder with a 0 - 90 % ethanol solution at room temperature. The results are shown in Fig. 1.

As shown in Fig. 1, although lipase or protease was not extracted at 25 % of ethanol concentration, almost of α-amylase was extracted together with α-AI at that concentration. When the ethanol concentration was raised to 50 %, α-amylase was also extracted by about 40 % although no change was observed for the extraction ratio of α-AI. When the ethanol concentration was raised to 60 %, α-amylase extraction ratio was decreased to about 3 % although the α-AI extraction ratio was slightly reduced to about 92 %. When the ethanol concentration was further raised, about 80 % of α-AI was extracted, while α-amylase was no more detected at 70 % of ethanol. Further, the α-AI extraction ratio was abruptly reduced at the ethanol concentration in excess of 80 %. In view of the above, the extraction for α-AI was determined as 58 - 70 % of ethanol solution capable of recovering α-AI at a high efficiency although the extract contains a little amount of α-amylase.

(2) Powderization condition of α-AI

α-AI can be powderized with no undesired effect on the α-AI activity by using the lyophilization method, but it results in a great increase of the production cost. Since α-AI is heat stable, use of spray-drying method capable of processing a great amount of α-AI in a short period of time was tried.

After removed ethanol from the extracts for α-AI with 58 - 70 % of ethanol using pressure reduction or the like, the resultant precipitates were removed with filtration or centrifugation. Then the fraction between 15 K and 50 K of molecular weight were collected by the method of ultra-filtration. The fractions were powderized by using an atomizing drier (YAMATO Co., DL-41). The results are shown in Table 1.

## Table 1: Change of α-amylase inhibitor by spray-drying

| Inlet tempe- rature (°C) | Exit tempe- rature (°C) | Processing performance (l/h)* | Residual α-AI activity (%)** | Recovery ratio (%)*** |
|---|---|---|---|---|
| 90 | 40 | 0,7 | 100 | 85 |
| 95 | 43 | 0,9 | 100 | 90 |
| 100 | 45 | 1,0 | 100 | 94 |
| 105 | 47 | 1,0 | 97 | 95 |
| 110 | 50 | 1,1 | 80 | 96 |
| 115 | 55 | 1,3 | 25 | 96 |
| 120 | 60 | 1,4 | 0 | 96 |

*:   Indicated by the amount of liquid supplied that can be dried per one hour.

**:   α-AI activity obtained by lyophilizing was assumed as 100%.

***: The weight of α-AI drying product obtained by lyophilizing was assumed as 100%.

As shown in Table 1, no change for the residual activity ratio of α-AI was observed upto the inlet temperature of 90 to 100 °C. However, it is necessary to lower the processing amount per hour or increasing the degree of pressure reduction upto the inlet temperature of 95 °C, which results in the reduction of the processing performance or the recovery rate. Accordingly, the conditions were set such that the inlet temperature was 100 - 105 °C, the exit temperature was at 45 - 47 °C and the pressure reduction degree was 30 - 40 mmHg, so that it scarcely gives effect on α-AI activity one liter of material can be processed per one hour and a relatively low pressure reduction can be employed.

(3) α-AI purity

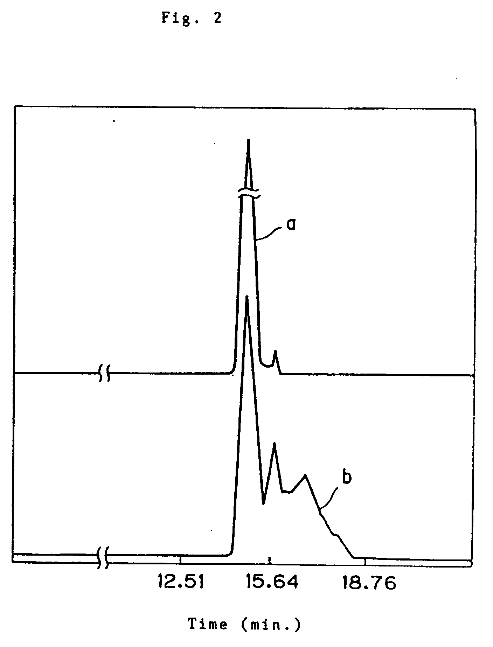

The purity of α-AI prepared by the process of the present invention was examined by a gel filtration method. Shodex G 520 column (for HPLC) was used for it and the eluting solution was 0,1 M Tris-HCl buffer (pH 7,2), the flow ratio was 1 ml/min, respectively. Detection was used absorbance at 210 nm. In the figure, "a" was prepared in accordance with the O'Connor's method, while "b" was prepared by the process in accordance with the present invention. The results are shown in Fig. 2. As shown in Fig. 2, α-Ai was eluted at the position of 18 min. The purity calculated from the peak height and the area of the substance was about 60 %.

In addition, enzymes for promoting decomposition and absorption of food such as α-amylase, lipase or protease were not observed at all in this fraction.

(4) Specific activity and recovery ratio of α-AI

The specific activity and the recovery ratio of α-AI prepared by the process according to the present invention and according to the method of O'Connor were examined in comparison. The results are shown in Table 2.

Table 2: Specific activity and recovery ratio of α-amylase inhibitor by the process of the present invention and the conventional process

| Fractions | Specific activity of α-AI (units/mg) | Recovery ratio* (%) |
|---|---|---|
| α-AI-1** | 7.560 | 40,5 |
| α-AI-2** | 3.900 | 88,3 |
| α-AI-3** | 3.780 | 85,7 |

*: The total α-AI activity in delipidated wheat powder assumed as 100 % (calculated).

**: α-AI-1 was prepared in accordance with the O'Connor's method, α-AI-2 was prepared in accordance with the process of the present invention and then lyophilized, α-AI-3 was prepared by the process according to the present invention and spray-dried.

The specific activity of α-AI-2 and α-AI-3 was about 50 % of α-AI-1, which depends on the purity of α-AI. When impurities of α-AI-2 and α-AI-3 were removed, the specific activity of them became equal with that of α-AI-1. Further, there was not difference observed the specific activities between lyophilization (no-heating, α-AI-2) and spray-dry (heating, α-AI-3).

The recovery ratio of α-AI-2 and α-AI-3 was 88 % and 85 %, respectively, it was higher than that of α-AI-1. This is suggested to be attributable to that the loss of α-AI could be kept to an extremely low level in the production process since the production step according to the present invention is extremely simple.

With the suggestion for the foregoing items, the following simple production process for α-AI was developed. After delipidating commercially available wheat powder (including from weak flour to strong flour) by using ethanol, water was added such that the final ethanol concentration was 58 to 70 %. The solution was stirred by way of a homomixer or the like at room temperature for 30 min and then extracts were obtained by centrifugation. The extraction procedure was repeated twice, these extracts were pooled and then removed ethanol by means of pressure reduction or the like. The resultant precipitates such as gultene were removed by filtration or centrifugation. The clear solution was filtrated by using ultra-filtration with a module of molecular weight 50 Kd. The permiates were then filtrated using the same way with a module of molecular weight 10 Kd. The resultant retentate was spray-dried under the conditions of the inlet temperature at 90 - 110 °C, exit temperature at 40 - 50 °C and the degree of pressure reduction of 30 - 40 mmHg, to obtain a powderized α-AI.

Wheat powder, other solvent and instruments used in the previous example were explained only for the illustration of the invention, which do not limit the starting materials, solvents and instruments.

As has been described above, according to the present invention α-AI not containing enzymes such as α-amylase and lipase can be obtained at a high α-AI recovery ratio by a simple step that consists of ethanol extraction and ultra-filtration.

## Claims

1. A method of extracting α-amylase inhibitor with 58 to 70 % ethanol from powderized products of wheat or the like.

2. A method of powderizing α-amylase inhibitor by using a spray-dry under the condition of the inlet

4

temperature at 90 to 110 ˚C and exit temperature at 40 to 60 ˚C.

Fig. 1

Fig. 2

Time (min.)